(19) Europäisches Patentamt
European Patent Office
Office européen des brevets

(11) Publication number: 0 103 000 B1

(12) EUROPEAN PATENT SPECIFICATION

(45) Date of publication of patent specification: 07.01.88

(51) Int. Cl.$^4$: C 12 Q 1/68, C 12 N 15/00, G 01 N 33/58

(21) Application number: 83900983.4

(22) Date of filing: 11.03.83

(86) International application number: PCT/SE83/00084

(87) International publication number: WO 83/03260 29.09.83 Gazette 83/23

# (54) METHOD FOR DETERMINATION OF TISSUE TYPES CODED BY MHC GENES.

(30) Priority: 11.03.82 DK 1055/82

(43) Date of publication of application: 21.03.84 Bulletin 84/12

(45) Publication of the grant of the patent: 07.01.88 Bulletin 88/01

(84) Designated Contracting States: AT BE CH DE FR GB LI LU NL SE

(56) References cited:
EP-A-0 062 286
EP-A-0 063 879
EP-A-0 084 796
WO-A-82/02060
ANN. REV. GENET. (1981), vol. 15, pages 169-187
Proc. Nat. Acad. Sci. USA, Vol. 77, No. 10, pp 6081-85, published October 1980 (PLOEGH H L ET AL) "Molecular cloning of a human histocompatibility antigen cDNA fragment"
Scand. J. Immunol. 14, pp 617-622, published 1981 (LARHAMMAR D ET AL) "Evolutionary relationship between HLA-DR antigen beta-chains, HLA-A, B, C antigen subunits and immunoglobulin chains"

(73) Proprietor: PETERSON, Per Arthur
Morkullvägen 29
S-752 52 Uppsala (SE)
(73) Proprietor: RASK, Lars
Säves Väg 14
S-752 63 Uppsala (SE)
(73) Proprietor: LERNMARK, Ake
Solosevej 11
DK-2820 Gentofte (DK)
(73) Proprietor: OWERBACH, David
Ermelundsvej 45
DK-2820 Gentofte (DK)

(72) Inventor: PETERSON, Per Arthur
Morkullvägen 29
S-752 52 Uppsala (SE)
Inventor: RASK, Lars
Säves Väg 14
S-752 63 Uppsala (SE)
Inventor: LERNMARK, Ake
Solosevej 11
DK-2820 Gentofte (DK)
Inventor: OWERBACH, David
Ermelundsvej 45
DK-2820 Gentofte (DK)

EP 0 103 000 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
Nature, Vol. 290, published 9 April 1981 (JORDAN B R ET AL) "Human HLA gene segment isolated by hybridization with mouse H-2 cDNA probes"

Proc. Nat. Acad. Sci. USA, Vol. 79, pp 1703-1707, published March 1982 (WIMAN K ET AL) "Isolation and identification of a cDNA clone corresponding to an HLA-DR antigen beta chain

Proc. Nat. Acad. Sci. USA, Vol. 79, pp 3687-3691, published June 1982 (LARHAMMAR D ET AL), "Complete amino acid sequence of an HLA-DR antigen-like beta chain as predicted from the nucleotide sequence: Similarities with immunoglobulins and HLA-A, -B, and -C antigens

Proc. Nat. Acad. Sci. USA, Vol. 79, pp 5966-5970, published October 1982 (STEILER D ET AL) "Isolation of a cDNA clone for the human HLA-DR antigen alpha chain by using a synthetic oligonucleotide as a hybridization probe

(74) Representative: Weisert, Annekäte, Dipl.-Ing. Dr.-Ing. et al
Patentanwälte Kraus Weisert & Partner Thomas-Wimmer-Ring 15
D-8000 München 22 (DE)

**Description**

The present invention relates to methods and means for the determination—or mapping—of human MHC tissue types, alternatively expressed, to methods and means for the determination of polymorphism in the MHC of man.

The term "MHC" means the "Major Histocompatibility Complex", also called the HLA-complex (Human Leukocytes system A). Both MHC and HLA-complex are generally accepted terms (see e.g. Clin. Immunol., Vol. 1 (1980), p. 63—80, Ed. Parker CW, WB Saunders Company).

The MHC is a genetic region present on human chromosome 6, that contains genes which code for products involved in many immunologic phenomena including allograft rejection and other cellular and humoral immune responses. The presently known HLA genes can be divided into two major categories: The HLA-A, B and C tissue antigens reflect the existence of at least three different loci which control the formation of glycoproteins (antigens) expressed on most nucleated cells. These antigens are referred to as *Class I* antigens. Another set of genes, exemplified by the HLA-D or DR (D-related) and DC genes (in the following collectively referred to as HLA-D genes), controls the formation of the so-called *Class II* antigens. These antigens have a restricted tissue distribution. Thus, Class II antigens differ from the Class I antigens inasmuch as they seem to appear mainly on cells of the immune system. Several complement components are also encoded in the MHC. These components constitute a third group of substances that are referred to as *Class III* molecules.

A schematic organization of the human MHC on the short arm of chromosome 6 is shown below:

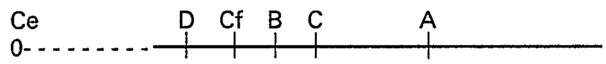


The centromere (Ce) is to the left. The A, B, C and other as yet undefined loci all code for class I antigen heavy chains while the D locus codes for α and β chains of DR, DC and other class II antigens.

Structure and tissue distribution of HLA antigens

Class I antigens, encoded in the A, B, and C loci of the MHC, appear on almost all nucleated cells. However, the quantitative expression varies drastically between different types of cells.

The structure of Class I antigens has been elucidated in great detail. All Class I antigens are composed of one heavy chain and one light chain. The heavy chain, encoded in the MHC, is a glycosylated polypeptide, which spans the plasma membrane and exhibits a portion of its COOH-terminal region on the cytoplasmic side of the membrane. The light chain of the Class I antigens is invariant regardless of which allelic product it is associated with. The light chain, also called $\beta_2$-microglobulin, is encoded by human chromosome 15 and consequently, its gene is not part of the MHC.

The knowledge of the protein structure, biosynthesis and genomic structure of Class II antigens is known in less detail than for the Class I antigens. However, Class II antigens are composed of two dissimilar, non-covalently associated, membrane-spanning glyco-polypeptides called α and β chains. The genes encoding these polypeptides are most likely part of the D-region of the MHC. So far, it has been established conclusively that both α and β chains of the DR and DC loci are encoded in the MHC.

The Class II antigens display a more restricted tissue distribution than the Class I antigens. Cells rich in Class II antigens are antigen-processing, dendritic cells, macrophages, antibody-producing B-lymphocytes and activated T-cells. In addition, Class II antigens occur on the surface of several types of epithelial cells.

Polymorphism of the HLA loci

The HLA antigens display an extensive genetic polymorphism at most loci although the genes controlling the complement components may be somewhat less polymorphic than the genes encoding Class I and II antigens. The HLA antigen polymorphism is reflected at the population level by the occurrence of a large number of HLA haplotypes. Each HLA haplotype represents a defined set of genes, all of which are inherited according to Mendelian laws. The multitude of HLA haplotypes is a consequence of the fact that virtually all MHC loci exhibit a large number of alleles. Allelic genes, although similar, differ from each other but occupy identical positions in different HLA haplotypes. A normal individual can only express two alleles at a given locus. However, the entire population may express a large number of alleles at such a locus. Presently, some 25 alleles have been identified at the HLA-A locus, a similar number at the B locus, about 10 at the C locus and a total of about 15 at the D locus (see Bodmer, W. F. Ed. Brit. Med. Bull. 34, 3 (1978)). Recombinant DNA techniques have revealed that β chains derived from the DR locus are genetically polymorphic while only a single α chain of the DR locus has been identified. In contrast both α and b chains of the DC locus appear to be genetically polymorphic.

A precise number of alleles cannot be given since new alleles are frequently discovered. Moreover, recently discovered alleles may turn out to be identical to previously detected ones as tedious, international collaborations have to be established to examine the specificity of the particular serological reagent used to define a new allele. Nonetheless, the high number of alleles and their frequency distribution in the population make it unusual to find two genetically unrelated individuals who share identical sets of alleles, *i.e.* who exhibit identical HLA haplotypes.

An allele consists of a segment of desoxyribonucleic acid (DNA) which comprises all the information needed to become expressed as a polypeptide chain. Thus, alleles differing in nucleotide sequences may give rise to different polypeptide chains. However, identical polypeptide chains may be derived from different alleles provided the nucleotide sequence differences are "silent" at the level of translation. Moreover, nucleotide sequence differences between alleles will not affect the polypeptide chain sequences provided the differences occur in introns or in untranslated portions of the exons. Consequently, alleles recognized as such at the DNA level may not emerge as alleles but as products of the same gene at the protein level. Despite this limitation of the polypeptide chain polymorphism, great numbers of alleles at various loci of the MHC have been discovered by serological analyses of the proteins derived from the alleles.

Some current indications for tissue typing

Tissue typing to identify products encoded by the MHC loci has proven to be of great importance in many areas of biomedicine. Originally, proteins encoded in the MHC were shown to be intimately involved in processes causing the rejection of transplanted tissues. The name "transplantation antigens" stems from this knowledge. It was soon realized that matching for MHC identity of the donor and the recipient was of predictive value in assessing the survival of a transplanted graft. In order to match the alleles indirect methods are used inasmuch as the proteins rather than the DNA were examined. This procedure is called tissue typing.

Interest in transplantation antigens increased further when it was realized that there exist a correlation between some MHC alleles and certain diseases. Thus, insulin-dependent diabetes is more prevalent among individuals with the alleles Dw3 and/or Dw4 while individuals with the allele Dw2 seem to be resistant to this disease (see Table I). Consequently, tissue typing may be used to predict, at the individual level, the relative risk to acquire a certain disease. This may be of importance e.g. in councelling individuals to avoid certain occupational hazards. Tissue typing is also of great value in forensic medicine, particularly in paternity cases, although the full value of tissue typing has not yet been realized. Finally, it has been observed that a correlation exists between some spontaneous abortions and the particular HLA haplotypes of the father and the mother.

Three examples of the value of tissue typing are described below in some detail:

1. Survival of *e.g.* transplanted bone marrow, kidneys and other organs is dependent on the HLA type of donor and recipient.

2) Survival of blood elements in blood transfusion is influenced by the HLA type of donor and recipient.

3) Susceptibility to develop certain diseases is related to HLA types.

Transplantation

HLA typing at the protein level of patients receiving *e.g.* kidney grafts revealed that graft survival was dependent on matching the HLA-types of the donor organ and the recipient. Transplantation between HLA-identical siblings was found to yield a higher incidence of kidney survival than transplantation between non-HLA-identical siblings. Recent results suggest that the matching for HLA-D results in a high success rate as regards accepted grafts. In fact, it has been found that survival of 90% of the grafts is obtained if the kidney donor and recipient have the same HLA-DR type. It is assumed that even minor differences in HLA-D is sufficient to initiate an immune response leading to rejection of the transplanted kidney. It should be pointed out that the immune response is complex and involves the activation of so-called T helper cells which, in class II antigen-dependent mechanisms, alert and stimulate the development of cytolytic lymphocytes and antibodies directed against the grafted tissue.

Blood transfusion

The transfusion of blood in certain blood cell deficiency diseases is also influenced by matching at the protein level for HLA-types. In certain thrombocyte disorders, the replacement therapy by transfusion often results in the formation of cytotoxic antibodies against the HLA antigens expressed on the surface of the thrombocytes. This reaction can be prevented by HLA-DR matching of donor and recipient blood.

Disease susceptibility

Many diseases have been studied to test the possibility that the appearance of a disease is related to a particular HLA-antigen. The frequency of an antigen in a population of individuals suffering from the disease is compared to the frequency of the same HLA antigen in a healthy control population. The degree of association between a given HLA antigen and the disease is expressed as the relative risk (RR). The relative risk indicates how many times more frequently, the disease occurs in a group of individuals carrying the particular antigen compared to the control population. A number of diseases shown to be related to a particular HLA antigen have been summarized in the table below. Several review articles are available on this subject (see Immunological Review, 70, 1—218, 1983).

TABLE I
Relationships between HLA and disease

| Disease | Associated HLA antigen | Frequency (%) of antigen | | Relative risk |
|---|---|---|---|---|
| | | Controls | Patients | |
| Ankylosing spondylitis | B27 | 8.6 | 89 | 90.1 |
| Reiter's syndrome | B27 | 8.6 | 77 | 35.9 |
| Acute anterior uveitis | B27 | 8.6 | 47 | 9.4 |
| Rheumatoid arthritis | Dw4 | 19.4 | 48 | 3.9 |
| Multiple sclerosis | Dw2 | 25.8 | 60 | 4.3 |
| Myasthenia gravis | B8 | 23.7 | 56 | 4.1 |
| Celiac disease | Dw3 | 26.3 | 96 | 73.0 |
| Dermatitis herpetiformis | Dw3 | 26.3 | 83 | 13.5 |
| Chronic autoimmune heptatitis | Dw3 | 26.3 | 71 | 6.8 |
| Sicca syndrome | Dw3 | 26.3 | 87 | 19.0 |
| Idopathic Addison's disease | Dw3 | 26.3 | 76 | 8.8 |
| Graves' disease | Dw3 | 26.3 | 61 | 4.4 |
| Insulin-dependent diabetes | Dw2 | 25.8 | 0 | 0.0 |
| | Dw3 | 26.3 | 48 | 2.5 |
| | Dw4 | 19.4 | 49 | 3.9 |
| Subacute thyroiditis | Bw35 | 13.1 | 72 | 16.8 |
| Psoriasis vulgaris | Cw6 | 33.1 | 88 | 14.9 |
| Idiopathic hemachromatosis | A3 | 26.9 | 73 | 7.4 |
| | B14 | 4.5 | 19 | 5.0 |
| C2 deficiency | Strongly associated with Dw2, in particular the A25, B18, Dw2 haplotype | | | |
| Congenital adrenal hyperplasia (21-hydroxylase deficiency) | Closely linked to HLA and associated with HLA-Bw47 | | | |

From Svejgaard and Ryder in Clinical Immunology (eds. F. H. Bach and R. A. Good), p. 173—181, Academic Press, N.Y. (1980).

According to the results presented in this patent application the development and manifestations of some such diseases may be considered related to the structure and expression of one or more genes in the MHC-encoding HLA antigens.

Current methods for tissue typing

Tissue typing, *i.e.* determination of the particular HLA alleles expressed on the surface of cells has hitherto been accomplished by indirect means since the protein derived from an allele rather than its DNA, has been examined. This is a tedious procedure and antisera reacting specifically against each transplantation antigen have to be available. In determining the expressed allele at some HLA loci homozygous typing cells are being used. It is easily realized that both types of methodology require reagents that frequently are of poor quality and hard to come by.

Currently, alleles of the A, B, C, DR and DC loci are identified by having specific antibodies react with the proteins derived from the alleles. The method used is called the "Lymphocyte Microcytotoxicity Test".

Thus, carefully selected antisera recognizing only one allelic protein of a given locus are placed in microtiter wells. Cells of the individual to be tissue typed are added to each well. After a period of incubation complement is added. Antibodies that have interacted with the cells will fix complement and the cells will lyse. By scoring which antisera have induced lysis, the tissue type can be determined. However, polypeptides derived from alleles not yet discovered will, of course, escape detection.

Alleles of the HLA-D loci are also being identified by a procedure called the "Mixed Lymphocyte Reaction". In that method homozygous typing cells are mixed and cultured together with the cells to be typed. Provided the class II antigens differ between the cells to be typed and the homozygous cells, proliferation will occur. Details of this and the above mentioned technique are excellently described in Clin. Immunol. (Vol. 1, Ed. Parker, C. W. pp. 67—89 (1980), W. B. Saunders Co.).

Some drawbacks of the presently employed techniques of tissue typing are:

1) The techniques are indirect.

2) Serological reagents that give unambiguous typing results are hard to come by and are difficult to standardize.

3) Prolonged storage of antisera and typing cells may reduce their usefulness.

4) Many reagents are not selective enough to identify only one single allele.

5) Alleles not yet identified will go undetected. Thus, it may be impossible to distinguish homozygosity from heterozygosity at a given locus if the heterozygous individual expresses an HLA antigen against which reagents are lacking.

6) Current techniques are tedious and cumbersome.

It is, thus, an object of the present invention to provide a simplified method for the determination of human MHC polymorphism which yields a much more refined determination than previous methods and is not subject to their limitations.

The European patent application EP—A—84796 (published on 3.8.83, filing date 11.1.83; priority date 22.1.82) discloses a HLA typing method based on restriction fragment length polymorphism which is said to be useful to make paternity or transplant or transfusion compatibility determinations or to diagnose diseases or to predict susceptibility to diseases. Said method is based on the use of cDNA hybridisation probes for the genes coding for Class II-alpha and Class II beta-chains.

The invention

The present invention provides a new method for determination of polymorphism of human MHC alleles for tissue typing, preferably of HLA-D tissue types. The method is characterized in that a probe which is able to hybridize to a base sequence in the MHC-locus coding for the beta-chain of a class II antigen is used in restriction enzyme mapping of DNA from different individuals, and that the size distribution of DNA fragments, hybridizing to the probe and originating from one first individual, is compared with the corresponding fragment distribution from a second individual or with the corresponding distribution reflecting a particular of polymorphism. The comparisons are carried out in order to determine a) the genetic matching of said other individual for donating tissues for the purpose of transplantation or blood transfusion to said first individual or b) the relative risk of the first individual to acquire a particular disease related to one or more of the alleles of the MHC or c) the biological paternity or maternity of the first individual or d) if the first and second individual exhibit the same polymorphism of one or more particular MHC allele, i.e. for use in forensic medicine. Specifically the comparisons mean that one is looking for the absence or presence of specific hybridizing fragment(s).

The method of the invention is based on analyzing the structure of the genes coding for or related to the HLA-D tissue types and is characterized in that the probe is selected from cDNA complementary to mRNA coding for the beta-chain of HLA-D (Class II) antigens, genomic DNA representing the actual genes (or gene complexes) containing the coding and non-coding sequences for Class II antigens and fragments thereof. In this aspect the distribution of DNA fragments from one individual may be compared e.g. according to a) and b) mentioned above.

Correspondingly, one preferred probe of the invention is characterized in that it is selected from cDNA complementary to the mRNA coding for the beta-chain of HLA-D (Class II) antigens, genomic DNA representing the actual genes (or gene complexes) containing the coding or non-coding sequences of Class II antigens, and fragments thereof.

Some probes for the beta-chain coding regions of the Class II antigens of the human MHC system are known in the prior art (see e.g. Scand. J. Immunol. *14* (1981) pp. 617—622), but their use in tissue typing has been neither disclosed nor suggested.

Isolation of DNA

Isolation of the DNA to be examined as regards its MHC polymorphism is accomplished according to well established procedures (Blin, N. and Stafford, D. W., Nucleic Acid Research 3 (1976), pp. 2303—2308. Preferably the DNA will be isolated from blood cells and other nucleated cells.

Restriction endonuclease-fragmentation of DNA

Restriction enzyme mapping of DNA is known *per se* and comprises the steps of 1) restriction enzyme digestion of human DNA, 2) size fractionation of the fragments thus obtained, and 3) detection of specific

DNA fragments by their hybridization to a nucleic acid probe. The isolated DNA is fragmented by use of one or more DNA-specific restriction endonucleases. Two or more restriction endo-nucleases can be used separately on aliquots of the same DNA preparation. Two or more restriction endonucleases can also be used for consecutive or simultaneous digestions of the same aliquot of DNA. Each enzyme may require its own particular conditions to effect complete digestion. Consequently, defined conditions for the digestions will vary with the enzyme or enzymes used. However, the general principles for the use of restriction endonucleases have been outlined in several review. articles (see Roberts, R. J. Nucl. Acid. Res. r. 117 (1982)). It can be stated that restriction endo-nuclease digestions of DNA are always carried out in aqueous solutions buffered to a pH in the range of 4 to 9. In most cases, but not in all, the temperature of the digestions range from 15° to 65°C.

A large number of restriction endonucleases are commercially available. Two groups of restriction endonucleases appropriate for use in the present procedure can be distinguished: 1) Restriction endonucleases recognizing a particular DNA sequence of four bases. 2) Restriction endonucleases recognizing a particular DNA sequence of six bases.

Each group of restriction endonucleases comprise several members which recognize different base sequences. The restriction endonucleases of group 1 will on average generate more DNA-fragments than the enzymes of group 2. However, the recognition of a specific sequence of bases for each enzyme implies that the number of DNA fragments generated by an enzyme will vary from that of another enzyme. Suitable restriction endonucleases to be used in establishing the particular alleles encompassed within the MHC region of an individual are: Eco RI, Pvu II, Bam HI, Bgl II, Pst I etc. Most likely, the list of suitable enzymes may be made much longer, particularly since new restriction endonucleases will be discovered in the future.

Separation of DNA-fragments

Restriction endonuclease digestions generate DNA fragments differing in molecular weights and, thus, in size. The molecular weights of the enzyme-generated DNA fragments are virtually perfectly correlated to each of the number of bases, phosphate groups and carbohydrate moieties of the DNA fragment. Size fractionation of the restriction endonucleases-generated DNA fragments involves a fractionation procedure taking advantage of the number of one or more of the above described constituents of the DNA fragments. The presently preferred way to separate DNA fragments generated by restriction endonuclease digestion is to use electrophoresis either with agarose or polyacrylamide as the support. However, fractionation of DNA fragments may also be accomplished by using other procedures such as gel chromatography, affinity separation techniques, etc.

The electrophoretic migration behaviour of restriction fragments visualized after hybridization will allow the determination of their molecular weights and, hence, the number of bases they contain. Of course, the precision of the molecular weight determinations of the restriction fragments will improve should a standard sample of well defined DNA fragments be run in parallel with the sample to be examined.

Probes

One type of probe that hybridizes with all or some alleles of the polymorphic MHC locus coding for the beta-chain of a class II antigen is initially prepared from a cDNA complementary to an mRNA molecule. The mRNA is usually enriched from cells that express substantial amounts of a polypeptide chain derived from an allele of the MHC locus coding for the constituent beta-chains of class II antigens. Enrichment of the mRNA of choice is carried out as described (Kvist, S. *et al.,* Cell, 29, 61—69, 1982).

The isolated mRNA fraction consists of many types of mRNA molecules. All the mRNA species are used as templates for reversed transcription generating double-stranded cDNA. The double-stranded cDNA molecules are separately ligated to a linearized plasmid which subsequently is used to transform a strain of E. coli. The transformed bacteria are cloned and those which support the replication of plasmids containing cDNA inserts corresponding to polymorphic alleles of the MHC are selected. The procedures are well known and outlined in US Patent Specification No. 4237224. The techniques described above frequently yield cDNA that corresponds only to a portion of the corresponding protein.

There are also other routes for preparing probes, *e.g.* synthetic routes for probes containing short base sequences. Once prepared a cDNA clone or a synthetic DNA fragment corresponding to part of that clone may be used for the isolation of genomic DNA flanked by parts of such coding DNA. Thus, it is possible to purify relevant chromosomal DNA, containing both coding and non-coding sequences, which then can be produced in large quantities according to US Patent Specification No. 4237224 and wholly or in parts be used as probes.

Concerning alleles of Class II antigen loci probes recognizing β chain alleles have to be used to establish Class II antigen polymorphism. Such probes can be obtained and they do not hybridize to alleles of the Class I antigen loci.

Probes derived from sequences unique to a certain allele may be used to detect only such DNA fragments that are derived from the particular allele. Available sequence information about Class II antigen genes indicate that allele-specific probes have to be short. Therefore, such probes are most easily prepared by automated nucleotide synthesis employing standard techniques (see Caruthers *et al.* Genetic

Engineering, vol. 3. p. 1—17 (1982) Plenum Press, New York). Using probes unique for an allele or for alleles of a single locus will simplify determinations of which alleles are present in a given sample of DNA. However, in many cases similar information will be obtained also with probes of less specificity provided appropriate restriction endonucleases are chosen for the digestions.

As outlined above allele-specific probes should be short. However, probes consisting of only few bases may hybridize also to DNA sequences not encompassed by the MHC. Therefore, probes consisting of between 100 and 1500 bases provide the most specific results. In fact, two probes in that size range corresponding to Class II antigens will yield hybridization patterns containing more information than serological analyses that have to be carried out with more than 15 antisera. Moreover, antisera are not available against some of the products of the alleles discovered by the mapping technique.

The probe to be used may preferably be covalently labeled with analytically indicatable compounds or groups by techniques known *per se*. Examples of such compounds are: enzymatically active substances, substrates for enzymes, co-factors, co-enzymes and fluorescent, luminescent and radioactive compounds. Examples of other substances not in themselves indicatable but possessing the property to react with analytically indicatable compounds, are: Protein A with the property of binding to labeled IgG (and *vice versa*), biotin with the property to bind labeled avidin (and *vice versa*), activated thiol compounds with the property to bind and react with thiol-containing analytically indicatable compounds (and *vice versa*) and, finally, haptens and antigens with the property to bind labeled antibodies (and *vice versa*).

Hybridization

Hybridization is defined as the formation of hydrogen bonds between the bases of a single-stranded DNA fragment and the complementary bases of a single-stranded DNA probe. The outcome of a hybridization is the formation of a double-stranded segment of DNA composed of the hybridizing DNA fragment and the DNA probe. It is obvious that hybridization, as defined here, does not require perfect complementarity between DNA fragment and probe but require that the complementarity is sufficient to allow the probe to bind in a selective manner to the DNA fragment.

To hybridize the probe to the restriction endonuclease fragments an aqueous solution containing the following components are mostly used: 1) One or more probes, 2) Formamide, and 3) Sodium chloride at a concentration of 0.1 to 1.5 M.

In addition, the solutions may contain Ficoll®, polyvinylpyrrolidone, bovine serum albumin and/or denatured, non-homologous DNA. The denatured, non-homologous DNA is being used to avoid non-specific sticking of the probe to sites on the paper that will bind any type of denatured DNA. When pre-hybridization is being used, the sheet to be exposed to the probe is treated with non-homologous, denatured DNA prior to hybridization with the probe. The hybridization solutions are buffered to pH values of 5 to 9 and the hybridizations are carried out at temperatures varying from 18° to 65° for 3 to 48 hours. The size of the DNA fragments that will hybridize to a probe is dependent on a variety of parameters among which are:

1. The restriction endonuclease or endonucleases used to generate the DNA fragments

For a given sample of DNA and for a given probe the pattern of hybridizing DNA fragments will vary with the specificity of the restriction endonuclease used. This, of course, is due to the particular sequence specificity of the enzyme. Different restriction enzymes will often generate fragments of different sizes from a single allele.

2. The particular alleles of the MHC in a sample to be analyzed

Using a given probe and a given enzyme different alleles of the polymorphic MHC locus will display fragments of different sizes that hybridize with the probe. Alternatively, some alleles may not share enough sequence similarity with the probe to hybridize to it while other alleles display strong hybridization due to nucleotide sequence identity with the probe.

3. The probe employed

For a given sample of DNA and one or more given restriction endonuclease, the fragments hybridizing to the probe will depend on the probe used inasmuch as two probes differing in their nucleotide sequences, but derived from the same cDNA or genomic fragment, may not hybridize to sequences present in the same restriction fragment.

The usefulness of the invention will be discussed below by demonstrating five examples from three important fields of application. These examples do not in any way serve to limit the invention and the average scholar will easily realize other fields of application not exemplified here. Although Class II-alpha chain probes are also quoted in the examples, the method claimed is limited to the use of Class II-beta chain probes.

Examples

All examples utilize a common technology which involves

a) extraction and isolation of genomic DNA

b) restriction enzyme digestions of the isolated DNA

c) separation of the restriction fragments by size

d) subjecting all of the separated DNA fragments to hybridization with at least one labelled probe derived from copy or genomic DNA of the MHC locus

e) identification of DNA fragments that have hybridized with the probe or probes

f) comparing the hybridization pattern recorded with those of one or more individuals.

Details of the experimental protocols pertinent to the examples described here are outlined below.

Blood cells

Blood (20 ml) was drawn from individuals which had been tissue typed for HLA-DR antigens with serological techniques.

Preparation of genomic DNA

In a typical preparation leucocytes were isolated from 20 ml of blood by centrifugation. The cells were suspended in 40 ml of 0.05 M Tris-HCl buffer, pH 7.5, containing 0.01 M NaCl and 0.001 M EDTA Sodium dodecyl sulfate (0.1 g in 0.5 ml of water) and proteinase K (Merck AG) (4 mg in 0.4 ml of water) were added. The sample was incubated at 37° under shaking for 16 h and thereafter it was extracted with phenol (40 ml) saturated with 20 mM Tris-Cl buffer, pH 7.5, containing 1 mM EDTA. After phase separation of centrifugation, the upper phase was extracted with 40 ml of water-saturated phenol:chloroform:isoamylic alcohol (25:24:1 v/v). The resulting upper phase was finally extracted with 20 ml of water-saturated chloroform:isoamylic alcohol (24:1 v/v). The upper phase was dialyzed three times against 2l-portions of 10 mM Tris-Cl buffer, pH 7.5, containing 1 mM EDTA for a total period of 24 h at +4°C. One-tenth the volume of 3 M sodium acetate buffer, pH 6.0, and 2.5 times the volume of cold ethanol were added to the dialyzed material. The DNA was precipitated in 250 ml glass tubes at −20°C for 12 h, and subsequently it was sedimented by centrifugation at 6000×g for 30 min at +4°C.

The DNA precipitate was dissolved in 10 mM Tris-Cl buffer, pH 7.5, containing 1 mM EDTA. Ethidium bromide was added to a final concentration of 800 µg/ml and CsCl to a final density of 1.397. The solution (total volume: 30 ml) was centrifuged for 40 h at +20°C in a Sorvall Ti 865 rotor at 41 000 rpm. Chromosomal DNA was thereafter recovered under ultraviolet light and was dialyzed against 2 l-portions of 10 mM Tris-Cl buffer, pH 7.5, containing 1 mM EDTA that were changed four times. The material was subsequently precipitated with ethanol and sodium acetate as described above. The precipitate was washed twice with ice-cold ethanol (70% v/v) in water, then lightly dried under vacuum, and finally carefully suspended in 300 µl of 10 mM Tris-Cl, buffer, pH 7.5, containing 1 mM EDTA.

Restriction enzyme digestion

Samples of DNA (10 µg) were separately digested with 10 units of restriction enzymes such as Bam HI, Pvu II, Eco RI and Bgl II (New England Biolabs) for 2 h at 37°C. The digestions were terminated by heating at 65°C for 2 min.

Electrophoresis

DNA-fragments from the digested samples were separated by electrophoresis in a 0.7% (w/v) agarose gel in 0.045 M Tris-acetate buffer, pH 8.3, at 50V for 12 h. The electrophoresis was carried out at room temperature in an Iceberg Design M 100 A apparatus. The DNA fragments were subsequently transferred onto nitrocellulose filters (BA 85 Schleicher and Schüll) according to the method described by Southern (J. Mol. Biol. 98, 503 (1975)). After transfer, the filter was dried under vacuum at 80°C for 2 h.

Isolation of probes corresponding to human class II transplantation antigens

Probes of the translated and 3′-untranslated parts of cDNA clones corresponding to HLA-DR and HLA-DC β chains respectively, were separately isolated by agarose gel electrophoresis. Probes corresponding to the translated portions of HLA-DR and HLA-DC α chains were purified by the same method. Fig. 1 gives a schematic picture of the different probes and the cDNA clones they were isolated from.

Radioactive labelling of the probes

The probes used were radioactively labelled with α[$^{32}$P]ATP by so called nick-translation (Rigby *et al.*, J. Mol. Biol. 113, 237—251 (1977)). The specific activities of the probes were approximately $3 \cdot 10^7$ cpm/µg DNA. Just before use, the probe, in a volume of 1 ml, was denatured by the addition of 0.5 ml of 0.2 M NaOH for 10 min at room temperature whereafter it was neutralized by adding 0.625 ml of 1.5 M sodium phosphate, pH 7.5 and put on ice.

Hybridization on nitrocellulose filters

The nitrocellulose filters were pre-hybridized for 3 h at 42°C in a solution containing formamide (50% v/v), dextran sulfate (1 g/ml), 0.9 M NaCl, 0.045 M sodium phosphate buffer, pH 7.5, 0.002 M EDTA, bovine serum albumin (1 mg/ml), ficoll (1 mg/ml), polyvinyl-pyrrolidone (1 mg/ml), 0.05 M Hepes, sodium dodecyl-sulfate (1 mg/ml) and single-stranded, sonicated salmon sperm DNA (80 µg/ml). The prehybridization solution was subsequently exchanged for the hybridization solution containing a

denatured, radioactively labelled probe (approximately $5 \cdot 10^6$ cpm) in 10 ml of a solution identical to the prehybridization solution except that the amounts of bovine serum albumin, Ficoll® and polyvinylpyrrolidone were reduced to one-fifth the concentration in the prehybridization solution. In addition, the concentration of Hepes was reduced to half that of the prehybridization solution. The hybridization of the probe was carried out at 42°C for 24 h.

The filters were subsequently washed according to the following protocol:

| | |
|---|---|
| 1. Twice at room temperature with a solution containing: | 0.36 M NaCl<br>0.018 M sodium phosphate, pH 7.5<br>0.001 M EDTA<br>1 mg/ml sodium dodecyl sulfate |
| 2. Twice for 15 min at 55°C with a solution containing: | 0.036 M NaCl<br>0.0018 M sodium phosphate, pH 7.5<br>0.0001 M EDTA<br>1 mg/ml sodium dodecyl sulfate |
| 3. Finally, once at room temperature with a solution containing: | 0.036 M NaCl<br>0.0018 M sodium phosphate, pH 7.5<br>0.001 M EDTA |

Autoradiography

The nitrocellulose filters were dried and placed in X-ray cassettes together with Kodak XAR-5 films and DuPont Cronex lightning-plus screens at −70°C for 3 days. The films were subsequently developed in an Agfa-Gevaert automatic developer.

Example 1

Serological tissue typing techniques are dependent on the availability of cells that can be used as reference. Such cells are usually homozygous at one or more of the polymorphic MHC loci. To demonstrate that the method of the invention is superior to presently used serological techniques DNA of homozygous typing cells was subjected to the protocol described above. The cells used were homozygous at the DR locus and displayed Class II antigens of specificities 1, 2, 3, 4, 5, 6, 7 and 8, respectively. No serological typing data for Class II antigens of the DC locus were available.

In most cases, it is not known whether antisera used for typing of Class II antigens contain antibodies directed against both or only one Class II antigen chain. However, since DR and DC α and β chain cDNA inserts have been isolated they were all used as probes in the hybridizations.

The cDNA of the DR α chain hybridized to a single band of identical size regardless of from which typing cell the DNA had been isolated provided the restriction endonucleases Eco RI or Pst I had been used for the digestion (not shown). However, when the digestions had been carried out with the enzyme Bgl II unique fragments hybridizing with the DR α chain probe was obtained when cells of DR-types 1, 3 and 6 were examined. For all other DR types the single hybridizing fragment was identical in size (see Fig. 2, lanes P—X).

Using the same DNA preparations digested either with the enzyme Bam HI or Pvu II hybridizations were carried out with a DC α chain probe. Fig. 2 shows that all eight cell types displaying different DR specificities gave rise to unique hybridization patterns. Since serological analyses only have revealed four DC antigens so far it can be concluded that the analyses shown here provide improved resolution. Moreover, the discovery of new DC α chain alleles is obvious.

DNA from the DR-homozygous cells was digested with the restriction endonuclease Pvu II and after size separation the DNA fragments were allowed to hybridize with DR and DC β chain probes, respectively. Fig. 3 depicts the results. It is obvious that the hybridization patterns were unique both as regards the cells and the probes used. Thus, the invention provides means to identify DR and DC β chain alleles and the method of the invention obviously resolves additional β chain alleles.

The data of Fig. 3 show that most DR and DC β chain alleles give rise to multiple fragments hybridizing with the cDNA probes used. This is an expected result since the hybridizing contiguous sequences of the probes correspond to genomic sequences of the alleles that are interrupted two or more times by intron sequences. Such introns may contain restriction enzyme sites but do not hybridize with the probes used. However, the observation of multiple DNA fragments in a single sample hybridizing with a probe may also indicate that the probe reacts with DNA fragments of more than one gene. Although the data of Fig. 3 clearly show that DR and DC β chain sequences, under the conditions used, only to a minor extent, if at all, cross-hybridize, the data require clarification. To this end the experiments of Fig. 3 were repeated and comparative analyses using DR and DC β chain probes corresponding to the translated portions of the cDNAs and the 3′-untranslated regions, respectively, were used (see Fig. 1). The DNA samples were digested with Bam HI prior to electrophoresis and hybridizations. Fig. 4 depicts the results obtained with the two DR β chain probes. It is evident that the untranslated probe did not give rise to unique hybridization patterns for each DR-type as did the translated probe, also when using this restriction enzyme. However,

when the DNA samples had been digested with the restriction enzyme PVU II the maps generated by the 3'-untranslated DR β chain probe were unique for each cell type differing in DR specificity (Fig. 5) but the number of restriction fragments hybridizing with this probe was less than those hybridizing with the DR β chain translated probe (see Fig. 5).

Fig. 6 shows restriction patterns of DNA from homozygous typing cells digested with the enzyme Bam HI and hybridized with probes corresponding to DC β chain translated and 3'-untranslated regions, respectively. The translated probe yielded unique restriction maps for each one of the cell types while the 3'-untranslated probe, in most instances hybridizing only to a single fragment, could not distinguish the alleles of five of the eight cell types (see Fig. 6, lanes J—N). Taken at face value these data may suggest that the human haploid genome contains but a single DC β chain gene.

The data presented in Figures 2—6 demonstrate that by using various β chain probes and restriction enzymes tissue typing can be performed by the method of the invention. Not only does this method allow the detection of new class II antigen (HLA-D) alleles, as exemplified by the data on the DC locus, but when α chain probes are concomitantly used, it also provides means for separate analyses of α and β chain alleles, something that so far has not been accomplished by serological techniques.

Example II

To examine the resolution of the method of the invention DNA was isolated from four individuals serologically typed to express the specificities DR 7/7 (one individual) DR 2/7 (two individuals) and DR 2/2 (one individual). All individuals examined were genetically unrelated. Aliquots of the samples were separately digested with the enzymes Pvu II and Bam HI, respectively, and restriction maps were established using translated probes of DR and DC β chain cDNA clones. The results are summarized in Fig. 7. As expected, the DR β chain probe gave distinct patterns for the two homozygous samples regardless of the enzyme used. However, a combined restriction map of the two homozygous individuals was identical to the maps recorded for the heterozygous individuals. Likewise, the DC β probe provided unique maps for the DR-homozygous individuals. However, the restriction maps of the two heterozygous individuals were dissimilar and only for one individual did the map correspond to the combined maps of the homozygous individuals. The DR and DC loci occur close to each other on chromosome 6.

Through this close linkage it is to be expected that a given DR allele will frequently occur together with a given DC allele. However, the data presented in Fig. 7 demonstrate that despite this close linkage the method of the invention can independently analyze the DR and DC loci.

Example III

Serological analyses as well as "mixed lymphocyte cultures" to assess the HLA-DR phenotype require great skill and the reproducibility of such analyses is usually less than acceptable. To examine the reproducibility of the method of the invention DNA was isolated from two pairs of monozygotic twins. Each sample was handled separately and after enzyme digestions, aliquots were subjected to restriction mapping. Fig. 8 shows that the restriction maps obtained were identical for each twin within a pair. Thus, neither the restriction enzyme digestions nor the probes used did artifactually distinguish the twins in each pair. The method of the invention is obviously very reproducible.

Example IV

A $^{32}P$-labeled DC β chain cDNA probe (previously termed pDR-β-1) (Wiman *et al.* Proc. Natl. Acad. Sci. USA 79, 1703—1707 (1982) and also Larhammar *et al* Scand. J. Immunol. 14, 617—622 (1981)) was utilized to study restriction endonuclease-digested DNA isolated from a total of 92 individuals. DNA-fragments of a lambda-phage were used as the molecular size markers.

The human DNA was digested with the restriction enzyme Eco RI and resulting fragments were separated by slab gel electrophoresis and subjected to the Southern hybridization technique.

The Eco RI digestion resulted in at least 8 different fragments (kb is kilobases):

| Approx. size |
|---|
| 20 kb |
| 13 kb |
| 11 kb |
| 9 kb |
| 6 kb |
| 4 kb |
| 3.5 kb |
| 2.2 kb |

The *6 kb*-fragment was present in *all* 92 individuals. The *11 kb*-fragment was highly, but not completely, invariable. The other fragments showed a variable appearance. The *9 kb*- and *2.2 kb*-fragments occurred paired, i.e. both fragments were either present or absent.

The 92 individuals showed 17 different maps of the DNA fragments hybridizing to the probe, none of

the maps covering 20% of the total number of individuals. A donor for organ transplantation or blood transfusion will be sought in the group showing the same map as the person in need of such treatment.

In this study 45 out of the 92 individuals were healthy controls, 31 were persons with insulin-dependent diabetes (IDDM) and 16 were diagnosed as non-insulin dependent diabetic patients (NIDDM). The segregation of the *9 kb/2.2 kb* sibling fragments is shown below.

| Number | Healthy controls | IDDM | NIDDM |
|---|---|---|---|
| with fragments | 22 | 3 | 8 |
| without fragments | 23 | 28 | 8 |
| % with fragments | 49% | 10% | 50% |

The analysis of the IDDM patients was confirmed in a duplicate analysis.

The significance of the present observation is that the IDDM patients lack the *2.2/9 kb* sibling fragments more often than the general population. A possible explanation is that the IDDM patients lack an EcoRI site in an allele encoding an HLA-DC β chain. It should be noted that the probe used was a DC β chain rather than a DR β chain. The lack of one or two restriction enzyme sites may influence the resulting molecule encoded by the gene.

The observation of a low frequency of the *2.2/9 kb* sibling fragments among IDDM patients is also dramatic from the point of view that nearly 100% of all IDDM-patients express either HLA-DR 3, HLA-DR 4 or HLA-DR 3/4. These alleles are present in no more than 60% of the healthy population.

In another study we examined the restriction fragments hybridizing to the probe when the DNA had been digested with BAM HI and Pst I, respectively. For reasons of comparison Eco RI digested DNA was also examined. The population consisted of 54 non-related individuals; healthy persons (n=25) and IDDM patients (n=29). All subjects were randomly selected to obtain an idea of the population frequency of the hybridizing fragments. None of the examined individuals had been typed for HLA-DR.

Compared to the healthy individuals the IDDM patients exhibited the expected decreased frequency of the hybridizing 2.2 and 9.0 kb Eco RI fragments ($p<0.01$) (see above). Also two Bam HI fragments of 3.2 and 3.7 kb, hybridizing to the DC β chain probe were less frequent in the DNA samples of the IDDM patients as compared to the healthy subjects ($p_c<0.001$; see Table II). The 5.8 kb Bam HI ($p_c<0.05$) and the 4.9 and 6.0 kb Pst I ($p_c<0.05$) fragments showed increased frequencies among the IDDM patients.

The differences in the restriction maps between the two groups may be explained by the preponderance of HLA-DR3 and/or DR4 alleles among the IDDM patients. However, since the probe used was a DC β-chain cDNA and the exact linkage between the HLA-DR and -DC loci is not known an umabiguous explanation to the data cannot be given as yet. Nonetheless, the decreased frequency of the Bam HI 3.7 kb fragment in DNA of the IDDM patients does not seem to be dependent on the negative association established for HLA-DR 2 and IDDM (see Table I) since DNA of HLA-DR2 homozygous typing cells do not display this Bam HI fragment (see Figure 6, lane B). Further analyses are obviously required to establish the exact origin of the 3.7 kb fragment (see below). However, suffice it to say that the Bam HI fragment of 3.7 kb is rarely observed when DNA of IDDM patients is examined while the same fragment occurs more frequently (30—40%) in DNA of healthy subjects, even if they express HLA-DR4.

DNA of randomly selected IDDM patients and healthy subjects did not differ in the frequency of the 18 kb PstI fragment. The different frequencies of this fragment in DNA of IDDM patients and healthy persons only seem to apply when the comparison is made between individuals displaying identical HLA-DR phenotypes.

Example V

It was tested whether the HLA-DC β-chain cDNA probe will hybridize to fragments of different sizes generated when DNA from healthy individuals and diabetic patients being either HLA-DR identical or randomly selected, are digested with restriction endonucleases. Among individuals serologically typed to be HLA-DR 4 and/or 3/4, insulin dependent diabetes mellitus (IDDM) patients showed an increased frequency of a Pst I 18 kb DNA fragment. A Bam HI 3.7 kb DNA fragment, frequent among healthy subjects (30—40%), was rarely detected in DNA of IDDM patients (0—2%).

DNA was isolated from 22 IDDM patients, diagnosed before the age of 18 at the Department of Pediatrics, University of Linköping, Sweden, expressing either HLA-DR3/- (n=5), DR4/- (n=8), or DR3/4 (n=9). Additional DNA samples of IDDM patients (n=25) expressing either HLA-DR3 (3/3 or 3/-) (n=5), HLA-DR (4/4 or 4/-) (n=7) or HLA-DR3/4 (n=13) and of healthy controls expressing HLA-DR3 (n=4), HLA-DR4 (n=9), or HLA-DR3/4 (n=13) were isolated from frozen, previously DR-typed lymphocytes.

TABLE II
The distribution of restriction fragments in randomly selected IDDM patients and controls

| Sequence size (kb) EcoRI | IDDM (n=29) | Controls (n=25) | p-value |
|---|---|---|---|
| 2.2 | 3 (10%) | 12 (48%) | 0.002[c] |
| 3.5 | 23 (79%) | 16 (64%) | NS |
| 4.0 | 26 (79%) | 20 (80%) | NS |
| 9.0 | 3 (10%) | 12 (48%) | 0.002[c] |
| 13.0 | 21 (73%) | 15 (60%) | NS |
| 20.0 | 25 (86%) | 14 (56%) | 0.01[a] |
| BamHI* | IDDM (n=29) | Controls (n=25) | |
| 3.2 | 3 (10%) | 15 (60%) | 0.0001[d] |
| 3.7 | 0 ( 0%) | 10 (40%) | 0.0001[d] |
| 5.8 | 23 (79%) | 10 (40%) | 0.004[b] |
| 6.2 | 3 (10%) | 10 (40%) | 0.01[a] |
| PstI** | IDDM (n=27) | Controls (n=25) | |
| 4.0 | 7 (26%) | 3 (12%) | NS |
| 4.9 | 21 (78%) | 9 (36%) | 0.003[b] |
| 5.1 | 10 (37%) | 18 (72%) | 0.01[a] |
| 5.4 | 4 (15%) | 12 (48%) | 0.01[a] |
| 6.0 | 26 (96%) | 16 (64%) | 0.004[b] |
| 6.3 | 18 (67%) | 7 (28%) | 0.006[b] |
| 18.0 | 23 (85%) | 18 (72%) | NS |

Differences between IDDM and controls (Fisher's exact test correcting for the number of fragments
[a]$p_c>0.05$; [b]$p_c<0.05$; [c]$p_c<0.01$; [d]$p_c<0.001$.

NS is not significant.

*3.2 vs. 3.4 kb, 4.0 vs, 4.3 kb and 10.0 vs 12.0 kb Bam HI sequences were not resolved; only sequences which varied between individuals are shown.

**PstI sequences 10, 7.2, and 6.5 have not been scored.

The hybridization patterns of enzyme digested DNA, isolated from randomly selected IDDM patients (n=29) and healthy controls (n=25), were also compared. All patients were diagnosed before they had reached the age of 30 and were treated with insulin. These patients as well as the healthy controls were all Caucasians. DNA samples were separately digested with 3 restriction endonucleases. DC β chain cDNA probe hybridized to 5—9 BamHI, 4—7 EcoRI and 4—8 PstI restriction fragments in the different individuals tested (Table II). BamHI sequences of 10 kb, 8.0 kb, 7.0 kb, and 5.0 kb and EcoRI sequences of 11 kb and 6.5 kb were present in all individuals examined; the other sequences were highly variable.

Figure 9 shows restriction fragments detected in 3 non-related HLA-DR3/4 healthy individuals after digestion with BamHI (lanes 1—3), EcoRI (lanes 4—6) and PstI (lanes 7—9), respectively. BamHI sequences of 3.7 and 12 kb, an EcoRI sequence of 20 kb and PstI sequences of 18 and 5.1 kb varied between the individuals.

The frequencies of the 3.7 kb BamHI and 18 kb PstI sequences differed between serologically typed HLA-DR identical IDDM and healthy individuals (Table IV). The PstI 18 kb sequence was present in increased frequency among IDDM patients expressing HLA-DR4 and DR3/4. Patients expressing the HLA-DR allele differed from controls even if the p-value was corrected for the number of PstI fragments observed ($p_c<0.01$). The frequency of the 3.7 kb BamHI sequence was clearly decreased in IDDM patients compared to controls ($p_c<0.01$). It was also noted that 6 out of the 8 HLA-DR3 and/or 4 positive individuals having the 3.7 kb sequence were also missing the 18 kb PstI fragment. These results demonstrate the existence of HLA-D region β-gene nucleotide sequences, which are associated with IDDM.

TABLE III

Variable and invariable (*) restriction fragments detected with the HLA-DC β-chain cDNA probe in the individuals tested

| | | | | | | | | | | | |
|---|---|---|---|---|---|---|---|---|---|---|---|
| Eco RI | 20 | 13 | 11* | 9.0 | 6.5 | 4.0 | 3.5 | 2.2 | kb | | |
| Bam HI | 12 | 10* | 8.0* | 7.0* | 6.2 | 5.8 | 5.0* | 4.3 | 3.7 | 3.2 | kb |
| Pst I | 18 | 10 | 7.2 | 6.5 | 6.3 | 6.0 | 5.4 | 5.1 | 4.9 | 4.0 | kb |

TABLE IV

Pst I 18 kb and Bam HI 3.7 kb restriction endonuclease fragments detected by the HLA-DC β-chain probe in HLA-DR typed IDDM and control individuals

| | | Pst I 18 kb | | | Bam HI 3.7 kb | | |
|---|---|---|---|---|---|---|---|
| | No. of individuals | No. of positive | % | p-value[a] | No. of positive | % | p-value[a] |
| HLA-DR3* | | | | | | | |
| IDDM | 10 | 4 | (40) | NS | 0 | (0) | NS |
| Controls | 4 | 2 | (50) | | 0 | (0) | |
| HLA-DR4* | | | | | | | |
| IDDM | 15 | 15 | (100) | $p<0.04$[b] | 0 | (0) | $p<0.04$[b] |
| Controls | 9 | 6 | (67) | | 3 | (33) | |
| HLA-DR3/4 | | | | | | | |
| IDDM | 22 | 21 | (96) | $p<0.02$[b] | 1 | (5) | $p<0.05$[b] |
| Controls | 13 | 8 | (62) | | 4 | (31) | |
| All individuals | | | | | | | |
| IDDM | 47 | 40 | (85) | $p<0.025$[b] | 1 | (2) | $p<0.002$[c] |
| Controls | 26 | 16 | (62) | | 7 | (27) | |

*Both IDDM and control individuals were selected to express only HLA-DR3 (3/3 or 3/-) and HLA-DR 4 (4/4 or 4/-), respectively.

[a]Fisher's exact test was used to test differences between IDDM and control individuals. Multiplying the p-values with the number of fragments observed resulted in the following, corrected p-values ($p_c$): [b]$p_c>0.05$, [c]$p_c<0.01$.

Legends to Figures

Fig. 1 Schematic picture of a Class II antigen molecule composed of disulfide-containing α and β chains (*middle*). The Class II antigen α and β chain cDNA probes and the restriction sites used to isolate them are indicated at the top and at the bottom of the figure. Their approximate lengths can be estimated from the lines demarkating every 100 bp. Both α chain probes correspond to the translated portion while both translated (TR) and 3'-untranslated (UT) probes of the β chain cDNA clones were used.

Fig. 2 Hybridizations of a DC α chain cDNA probe (see Fig. 1) to DNA of individuals serologically typed to be homozygous for the DR specificities 1 to 8, respectively (lanes A—P). The DNA was digested separately with Bam HI (lanes A—H) and Pvu II (lanes I—P). The same DNA was also digested with Bgl II and hybridized with a DR α chain cDNA probe (lanes Q—X). DR 1 homozygous DNA occurs in lanes A, I and Q and DR 8 homozygous DNA in lanes P, H and X.

Fig. 3 Hybridizations of DR (A) and DC (B) β chain translated probes to DNA of individuals serologically typed to be homozygous for the DR specificities 1 to 8 (as noted above each lane). The figures denote the positions of markers of different sizes (expressed as kilobases, kb).

Fig. 4 Hybridizations of DR β chain cDNA translated (lanes A—H) and 3'-untranslated (lanes I—P) probes to DNA of individuals serologically typed to be homozygous for the DR specificities 1 to 8 (lanes A and I depict DR 1 DNA while lanes H and P depict DR 8 DNA). The DNA was digested with Bam HI.

Fig. 5 An experiment identical to that of Fig. 4 but the aliquots of DNA were digested with Pvu II rather than with Bam HI.

Fig. 6 Hibridization of DC β chain cDNA translated (lanes A—H) and 3'-untranslated (lanes I—P) probes as described for Fig. 4. The DNA was cleaved with Bam HI.

Fig. 7 Hybridizations of DR (lanes 1—8) and DC (lanes 9—16) β chain cDNA translated probes to DNA of two individuals phenotypically homozygous at the DR β chain locus (DR 7/7 in lanes 1, 5, 9 and 13; and DR 2/2 in lanes 4, 8, 12 and 16) and to DNA of two individuals serologically typed to express the DR specificities

2/7 (lanes 2, 6, 10 and 14 display the restriction patterns of one of the heterozygous individuals while the patterns of the other individual are depicted in lanes 3, 7, 11 and 15). The aliquots of DNA were separately digested with Pvu II (lanes 1—4 and 9—12) and Bam HI (lanes 5—8 and 13—16).

Fig. 8 Hybridization of DC (lanes 1, 2, 5, 6, 9, 10, 13 and 14) and DR (lanes 3, 4, 7, 8, 11, 12, 15 and 16) β chain cDNA translated probes to DNA of two pairs (AA and BB, respectively) of monozygotic twins. Lanes 1 to 8 depict DNA of the AA twins and lanes 9—16 show DNA of the BB twins. DNA in lanes 1 to 4 and 9 to 12 was digested with Pvu II while DNA in lanes 5—8 and 13—16 was cleaved with Bam HI.

Fig. 9 Hybridization of a DC β chain cDNA translated probe to DNA of three healthy and genetically unrelated individuals. The individual DNA preparations were separately digested with Bam HI (lanes 1—3), Eco RI (lanes 4—6) and Pst I (lanes 7—9).

**Claims**

1. A method for the determination of human MHC polymorphism in tissue typing for the purpose of organ transplantation, blood transfusion, for the use in forensic medicine, for paternal and maternal ascertainment or for assessing the relative risk for an individual to acquire a disease related to certain MHC alleles, characterized in that a nucleic acid probe, which hybridizes to a base sequence in a MHC-locus coding for the beta-chain of a class II antigen, is used in restriction enzyme mapping, and that the size distribution of DNA fragments, hybridizing to the probe and originating from one first individual, is compared with the corresponding fragment distribution from a second individual or with the corresponding distribution for a particular type of polymorphism, either of said comparisons being carried out in order to determine: a) the genetic suitability of said other individuals for donating tissues for the purpose of transplantation or blood transfusion to said first individual, b) the relative risk of said first individual to acquire a particular disease related to one or more MHC alleles, c) the paternity or maternity of said first individual or d) if the first and second individual exhibit the same polymorphism of one or more particular MHC allele, *i.e.* for use in forensic medicine.

2. A method according to claim 1 characterized in that the probe is selected from a) cDNA complementary to the mRNA coding for the beta-chain of Class II gene products (antigens) b) genomic DNA representing the actual Class II beta-chain genes and/or their flanking base sequences and c) fragments thereof.

3. A method according to claim 2 characterized in that the probe is complementary to any part of the mRNA coding for the beta-chain of a Class II antigen.

4. A method according to claim 1 or 2, characterized in that human DNA is treated with one or more restriction endonucleases resulting in the DNA fragments which are separated according to molecular size, preferably by gel electrophoresis, and transferred to a sheet binding DNA, preferably a nitrocellulose sheet, whereafter the filter is exposed to the probe and binding of the probe to complementary base sequences of DNA fragments of different sizes are identified.

5. A method according to claim 4, characterized in that the fragment distribution is examined for the appearance of fragments, the absence or presence of which is indicative of the relative risk of an individual to acquire a disease related to one or more MHC allele.

6. A method according to claims 3 or 4, characterized in that the restriction enzyme is Eco RI, and that the fragment distribution is examined for the appearance of two paired fragments of 9 kb and 2.2 kb, the absence of which in an individual expressing the serologically defined HLA-DR4 or DR3/4 indicates that said individual has an increased relative risk to acquire insulin-dependent diabetes mellitus.

7. A method according to claims 3 or 4, characterized in that the restriction enzyme is Bam HI, and that the fragment distribution is examined for the appearance of a fragment of 3.7 kb, the absence of which in an individual expressing the serologically defined HLA-DR4 or DR3/4 indicates that said individual has an increased relative risk to acquire insulin-dependent diabetes mellitus.

8. A method according to claims 3 or 4, characterized in that the restriction enzyme is Pst I, and that the fragment distribution is examined for the appearance of a fragment of 18 kb, the presence of which in individuals expressing the serologically defined HLA-DR4 or DR 3/4 indicates that said individual has an increased relative risk to acquire insulin-dependent diabetes mellitus.

9. A method according to claim 1 or 2, characterized in that DNA from each individual of a selected population preferably a family, is mapped, and the maps are correlated to the occurrence of a certain disease in said population to determine a relationship between the presence or absence of specific fragments in the maps of the disease affected and healthy persons.

10. A method according to any of the claims 1—9, characterized in that the nucleic acid probe used contains 100—1 500 bases.

**Patentansprüche**

1. Verfahren zur Determinierung von humanem MHC-Polymorphismus bei der Gewebebestimmung für den Zweck der Organtransplantation, der Bluttransfusion, für die Verwendung in der forensischen Medizin, für die Bestimmung der Vaterschaft und Mutterschaft, oder für die Festlegung des relativen Risikos eines Individuums, eine Krankheit, die mit bestimmten MHC-Allelen in Zusammenhang steht, zu

bekommen, dadurch gekennzeichnet, daß eine Nucleinsäureprobe, welche zu einer Basensequenz in einem MHC-Lokus hybridisiert, der für die β-Kette von Klasse-II-Antigen codiert, bei einer Bankaufstellung mittels Restriktionsenzymen verwendet wird und daß die Größenverteilung der DNA-Fragmente, die zu der Probe hybridisieren und von dem ersten Individuum stammen, mit der entsprechenden Fragmentverteilung von einem zweiten Individuum oder mit der entsprechenden Verteilung für einen besonderen Typ von Polymorphismus verglichen wird, wobei die Vergleiche entweder durchgeführt werden:

a) um die genetische Eignung der anderen Individuen für die Spende von Geweben, für den Zweck der Transplantation oder der Bluttransfusion für das erste Individuum festzustellen,

b) um die relative Gefahr des ersten Individuums, eine Krankheit zu bekommen, die mit einem oder mehreren MHC-Allelen in Zusammenhang steht, festzustellen,

c) um die Vaterschaft oder Mutterschaft von dem ersten Individuum zu bestimmen oder

d) für die Verwendung in der forensischen Medizin, wenn das erste und das zweite Individuum den gleichen Polymorphismus von einem oder mehreren MHC-Allelen zeigen.

2. Verfahren nach Anspruch 1, dadurch gekennzeichnet, daß die Probe ausgewählt wird unter

a) cDNA, welche zu der mRNA komplementär ist, die für die β-Ketten der Klasse-II-Genprodukte (Antigene) codiert,

b) genomischer DNA, die die tatsächlichen Klasse-II-β-Ketten-Gene repräsentiert und/oder ihren flankierenden Basensequenzen und

c) ihren Fragmenten.

3. Verfahren nach Anspruch 2, dadurch gekennzeichnet, daß die Probe zu irgendeinem Teil der mRNA, welche für die β-Kette des Klasse-II-Antigens codiert, komplementär ist.

4. Verfahren nach Anspruch 1 oder 2, dadurch gekennzeichnet, daß die humane DNA mit einem oder mehreren Restriktionsendonukleasen, welche DNA-Fragmente ergeben, behandelt wird, wobei die DNA-Fragmente entsprechend der Molekülgröße, bevorzugt durch Gel-Elektrophorese, getrennt und auf ein Blatt, welches DNA bindet, bevorzugt ein Nitrocelluloseblatt, übertragen werden, wonach das Filter der Probe ausgesetzt wird und die Bindung der Probe an komplementäre Basensequenzen der DNA-Fragmente unterschiedlicher Größen identifiziert wird.

5. Verfahren nach Anspruch 4, dadurch gekennzeichnet, daß die Fragmentverteilung für das Auftreten von Fragmenten, deren Anwesenheit oder Abwesenheit als Anzeichen für die relative Gefahr eines Individuums gilt, eine Krankheit zu erlangen, die mit einem oder mehreren MHC-Allelen in Zusammenhang steht, geprüft wird.

6. Verfahren nach einem der Ansprüche 3 oder 4, dadurch gekennzeichnet, daß das Restriktionsenzym Eco RI ist und daß die Fragmentverteilung auf das Auftreten von zwei gepaarten Fragmenten von 9 kb und 2,2 kb geprüft wird, deren Abwesenheit in einem Individuum, welches die serologisch definierte HLA-DR4 oder DR3/4 ausdrückt, anzeigt, daß für das Individuum eine erhöhte relative Gefahr besteht, insulinabhängige Diabetes Mellitus zu bekommen.

7. Verfahren nach einem der Ansprüche 3 oder 4, dadurch gekennzeichnet, daß das Restriktionsenzym Bam HI ist und daß die Fragmentverteilung für das Auftreten eines Fragments von 3,7 kb geprüft wird, dessen Abwesenheit in einem Individuum, welches serologisch definierte HLA-DR4 oder DR3/4 ausdrückt, anzeigt, daß das Individuum einer erhöhten relativen Gefahr unterliegt, insulinabhängige Diabetes Mellitus zu bekommen.

8. Verfahren nach einem der Ansprüche 3 oder 4, dadurch gekennzeichnet, daß das Restriktionsenzym PstI ist und daß die Fragmentverteilung auf das Auftreten eines Fragments von 18 kb untersucht wird, dessen Anwesenheit in Individuen, welche serologisch definierte HLA-DR4 oder DR 3/4 ausdrücken, anzeigt, daß das Individuum einer erhöhten relativen Gefahr unterliegt, insulinabhängige Diabetes Mellitus zu bekommen.

9. Verfahren nach einem der Ansprüche 1 oder 2, dadurch gekennzeichnet, daß die DNA von jedem Individuum einer ausgewählten Population, bevorzugt einer Familie, für eine Bankanlegung verwendet wird un die Banken zu dem Auftreten einer bestimmten Krankheit in der Population korreliert werden, um eine Beziehung zwischen der Anwesenheit oder Abwesenheit spezifischer Fragmente in den Banken der betreffenden Krankheit und gesunder Patienten zu bekommen.

10. Verfahren nach einem der Ansprüche 1 bis 9, dadurch gekennzeichnet, daß die verwendeten Nucleinsäureproben 100 bis 1500 Basen enthalten.

**Revendications**

1. Procédé pour déterminer un polymorphisme du MHC human dans la détermination du type de tissus en vue d'une transplantation d'organe, d'une transfusion sanguine, en médecine légale, pour vérifier une paternité et une maternité ou pour évaluer le risque relatif pour un individu d'acquérir une maladie reliée à certains allèles du MHC, caractérisé en ce qu'on utilise une sonde d'acide nucléique qui s'hybride à une séquence de bases dans un locus du MHC codant pour la chaîne béta d'un antigène de classe II dans la détermination de la carte de l'ADN de différents individus par des enzymes de restriction, et que l'on compare la distribution des dimensions des fragments d'ADN, s'hybridant à la sonde et provenant d'un premier individu, à la distribution des fragments correspondants, provenant d'un second individu ou à la

distribution correspondante obtenue pour un type de polymorphisme particulier, l'une ou l'autre de ces comparaisons étant effectuées pour déterminer:

a) l'aptitude génétique de ces autres individus à donner des tissus pour une transplantation ou du sang pour une transfusion au premier individu;

b) le risque relatif du premier individu à acquérir la maladie particulière reliée à un ou plusieurs allèles du MHC;

c) la paternité ou la maternité du premier individu, ou

d) si le premier et le second individus manifestent le même polymorphisme d'un ou plusieurs allèles particuliers du MHC, c'est-à-dire que cela est utile en médecine légale.

2. Procédé suivant la revendication 1, caractérisé en ce que la sonde est choisie parmi:

a) un ADNc complémentaire de l'ARNm codant pour la chaîne béta des produits génétiques de classe II (antigènes);

b) l'ADN génomique représentant les génes réels de la chaîne béta de classe II et/ou leurs séquences de bases disposées à côté et,

c) des fragments de ceux-ci.

3. Procédé suivant la revendication 2, caractérisé en ce que la sonde est complémentaire d'une quelconque partie de l'ARNm codant pour la chaîne béta d'un antigène de classe II.

4. Procédé suivant la revendication 1 ou 2, caractérisée en ce que l'ADN humain est traité avec une ou plusieurs endonucléases de restriction, ce qui fournit des fragments d'ADN qui sont séparés en fonction de la dimension moléculaire, de préférence par électrophorèse sur gel, et transférés sur une feuille fixant l'ADN, de préférence une feuille de nitrocellulose, et ensuite, le filtre est exposé à la sonde et les liaisons de la sonde aux séquences de bases complémentaires des fragments d'ADN de différentes dimensions sont identifiées.

5. Procédé suivant la revendication 4, caractérisé en ce que la distribution des fragments est examinée pour rechercher l'apparition des fragments dont l'absence ou la présence constitue une indication du risque relatif d'un individu à acquérir une maladie reliée à un ou plusieurs allèles du MHC.

6. Procédé suivant la revendication 3 ou 4, caractérisé en ce que l'enzyme de restriction est EcoRI et que la distribution des fragments est examinée pour rechercher l'apparition de deux fragments appariés de 9 kb et 2,2 kb, dont l'absence chez un individu exprimant l'HLA-DRK ou DR3/4 définis sérologiquement, indique que cet individu à un risque relatif accrû d'acquérir un diabète sucré insulino-dépendant.

7. Procédé suivant la revendication 3 ou 4, caractérisé en ce que l'enzyme de restriction est BAM HI et que la distribution des fragments est examinée pour rechercher l'apparition d'un fragment de 3,7 kb, dont l'absence chez un individu experimant les HLA-DR 4 ou DR 3/4 définis sérologiquement, indique que cet individu a un risque relatif accrû d'acquérir un diabète sucré insulino-dépendant.

8. Procédé suivant la revendication 3 ou 4, caractérisé en ce que l'enzyme de restriction est Pst I et que la distribution des fragments est examinée pour rechercher l'apparition d'un fragment de 18 kb, dont la présence chez des individus exprimant les HLA-DR4 ou DR 3/4 définis sérologiquement, indique que ces individus ont un risque relatif accrû d'acquérir un diabète sucré insulino-dépendant.

9. Procédé suivant les revendications 1 ou 2, caractérisé en ce qu'on détermine la carte de l'ADN de chaque individu d'une population choisie, de préférence une famille, et que l'on met ces cartes en corrélation avec l'apparition d'une certaine maladie dans cette population pour déterminer une relation entre la présence ou l'absence des fragments spécifiques dans les cartes des personnes en bonne santé et celles des personnes souffrant de la maladie.

10. Procédé suivant l'une quelconque des revendications 1 à 9, caractérisé en ce que la sonde d'acide nucléique utilisée contient 100 à 1 500 bases.

Figure 1

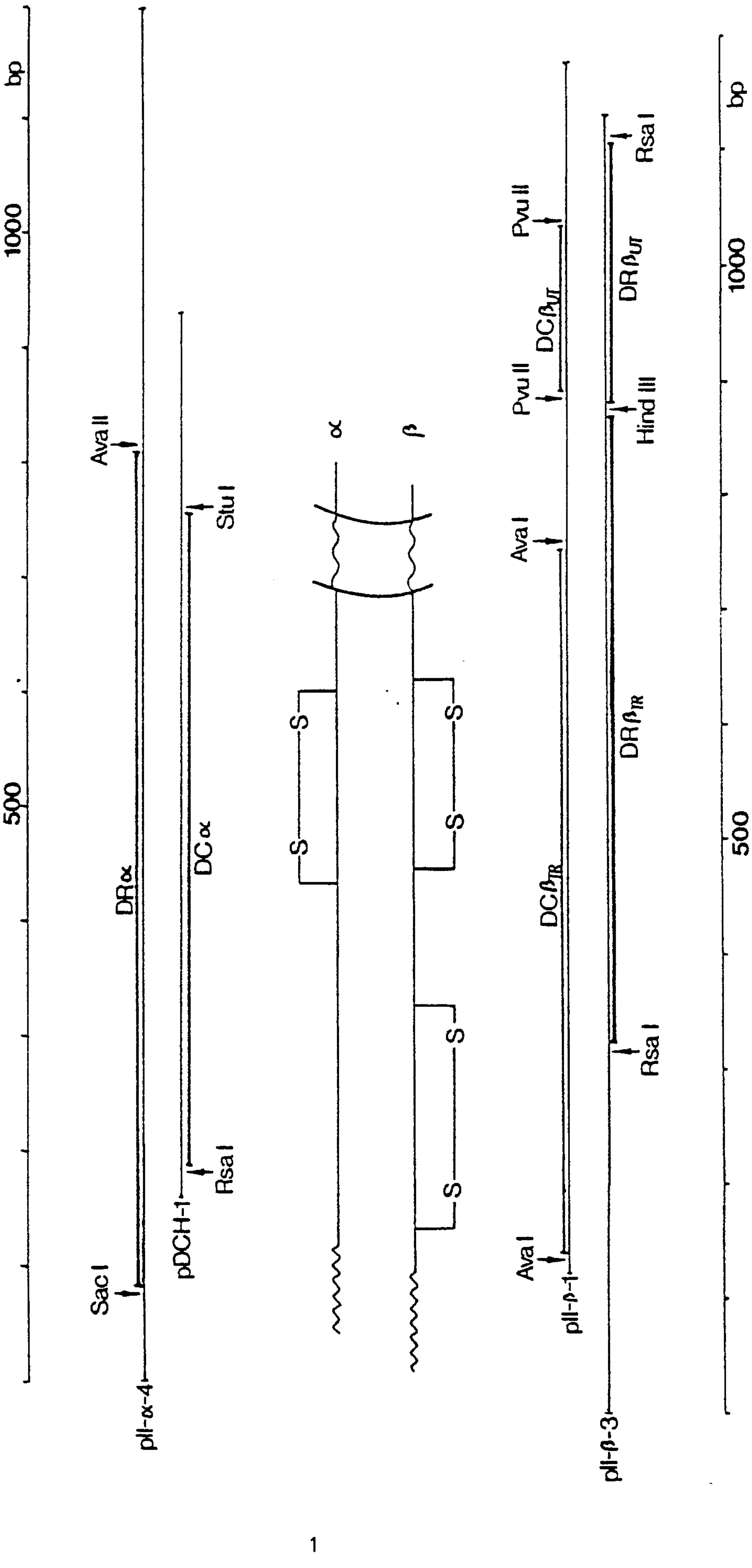
bp
1000
500
pII-α-4
Sac I
DRα
Ava II
pDCH-1
Rsa I
DCα
Stu I
α
β
S
S
S
S
S
S
pII-β-1
Ava I
DCβ$_{TR}$
Ava I
Pvu II
DCβ$_{UT}$
Pvu II
pII-β-3
Rsa I
DRβ$_{TR}$
Hind III
DRβ$_{UT}$
Rsa I
bp
500
1000

Fig. 2

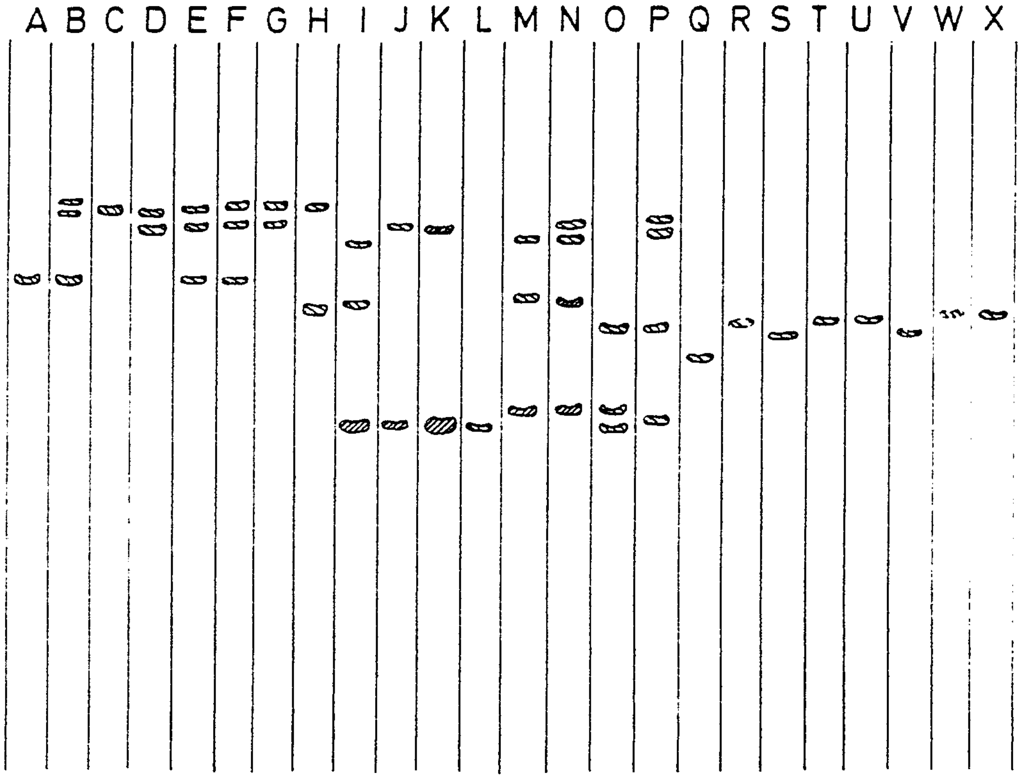
A B C D E F G H I J K L M N O P Q R S T U V W X

Fig. 3

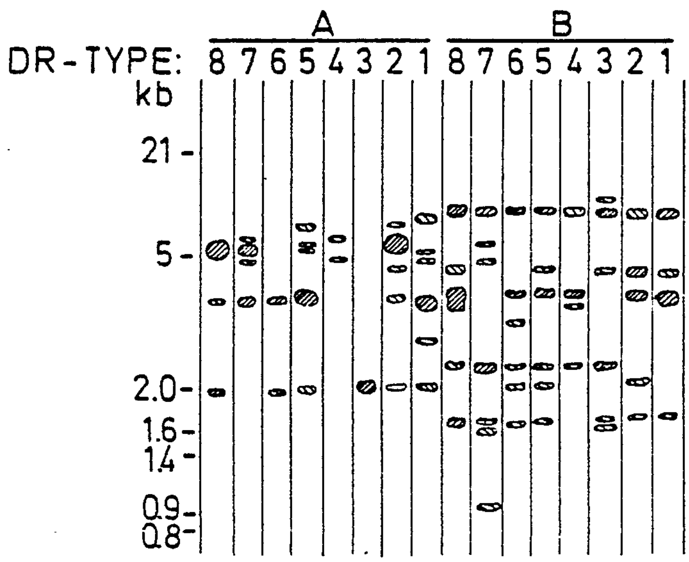
A
B
DR-TYPE: 8 7 6 5 4 3 2 1 8 7 6 5 4 3 2 1
kb
21
5
2.0
1.6
1.4
0.9
0.8

Fig. 4

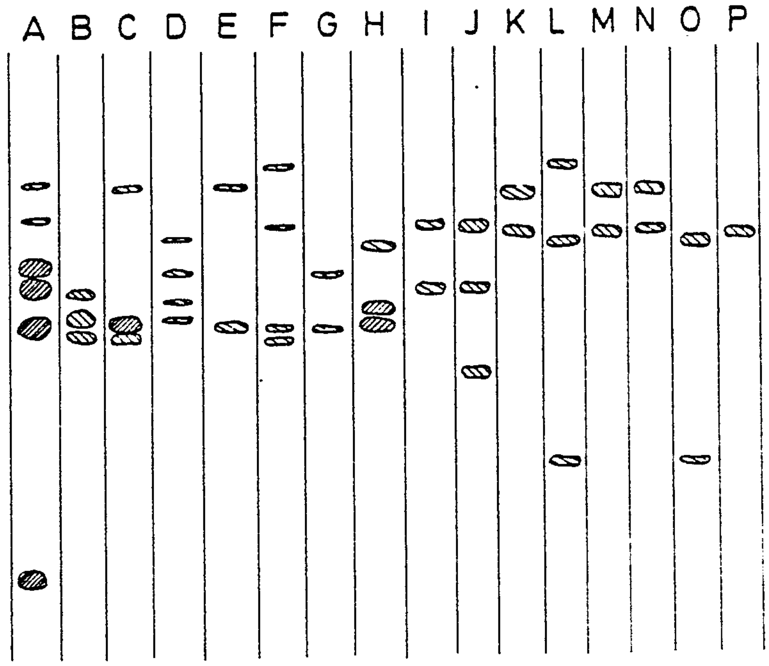


Fig. 5

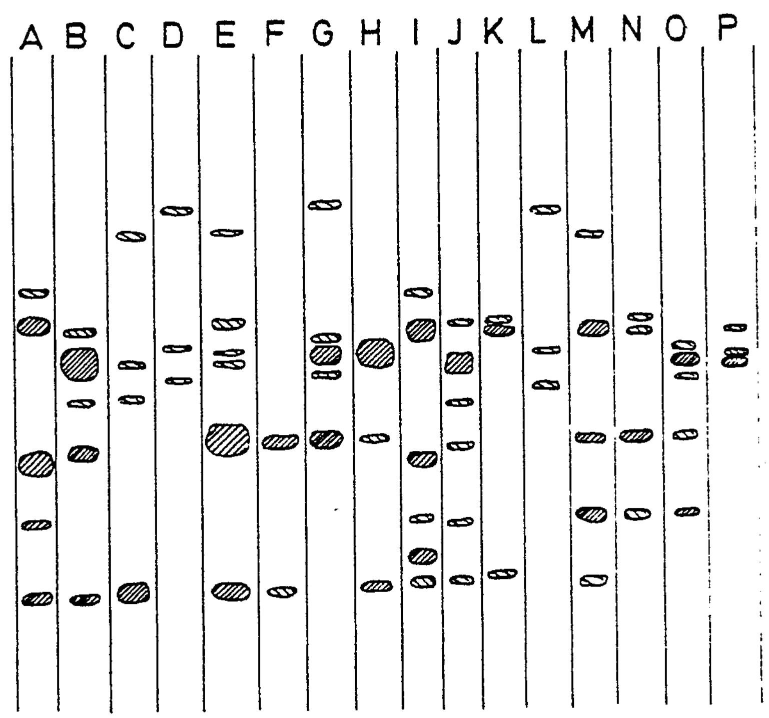

Fig. 6

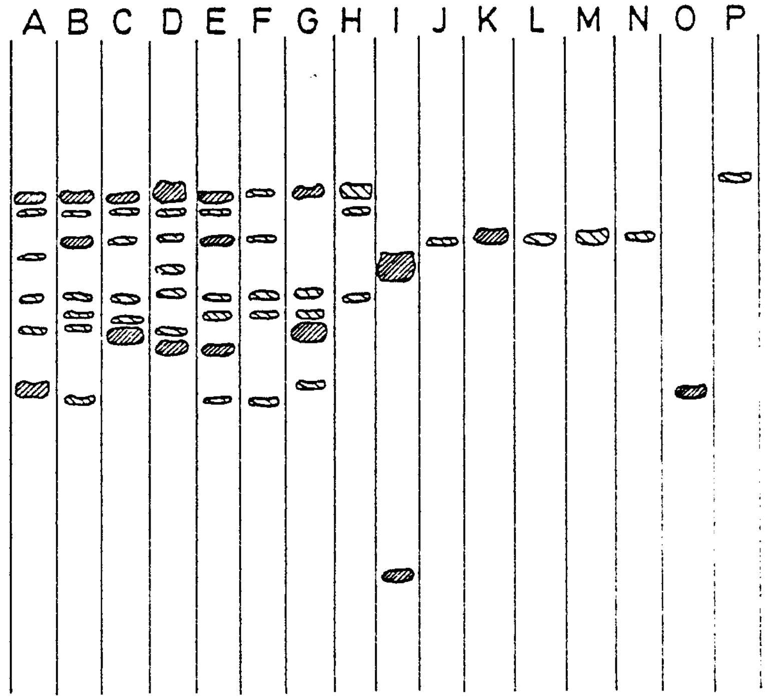
A B C D E F G H I J K L M N O P

Fig. 7

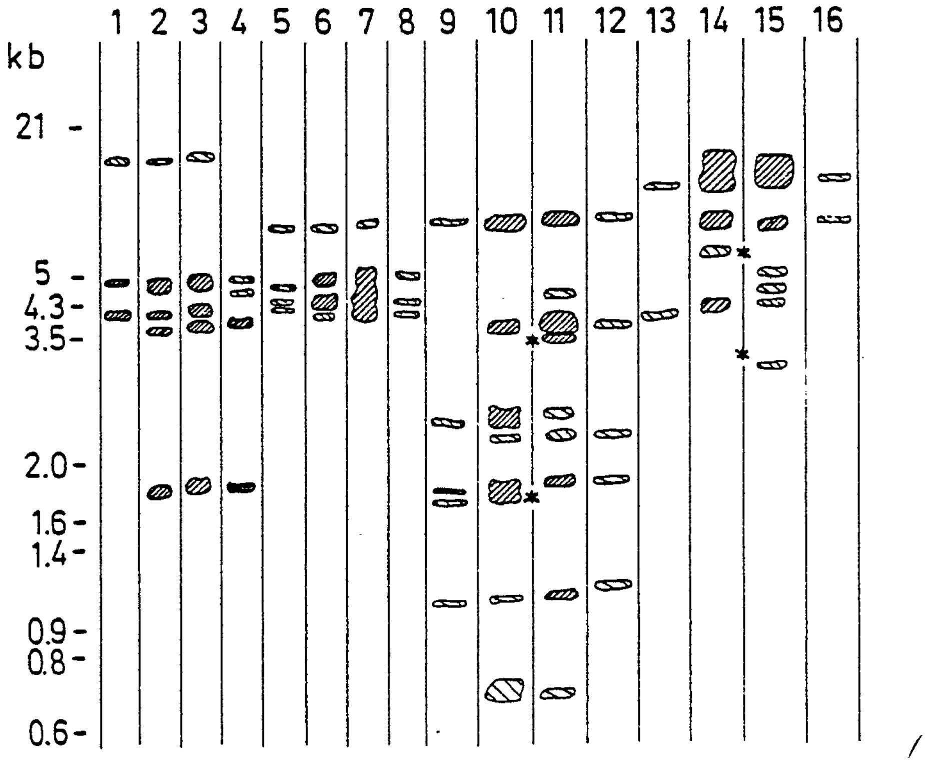
1 2 3 4 5 6 7 8 9 10 11 12 13 14 15 16
kb
21
5
4.3
3.5
2.0
1.6
1.4
0.9
0.8
0.6

Fig. 8

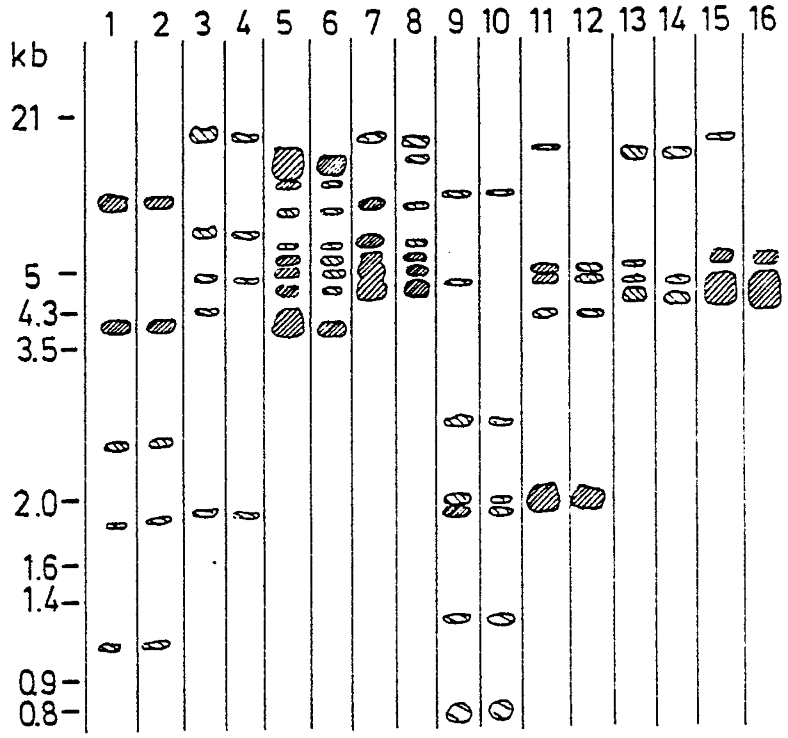
1 2 3 4 5 6 7 8 9 10 11 12 13 14 15 16
kb
21
5
4.3
3.5
2.0
1.6
1.4
0.9
0.8

Fig. 9

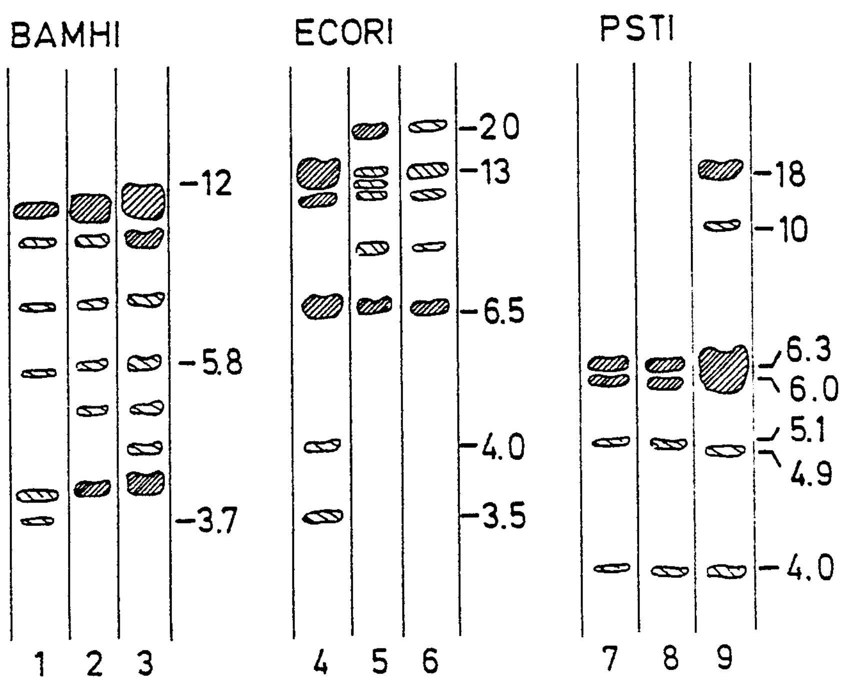
BAMHI
ECORI
PSTI
12
5.8
3.7
20
13
6.5
4.0
3.5
18
10
6.3
6.0
5.1
4.9
4.0
1 2 3
4 5 6
7 8 9